# EUROPEAN PATENT SPECIFICATION

(11) **EP 1 505 949 B1**
(45) Date of publication and mention of the grant of the patent: **07.02.2007**
(21) Application number: 03720518.4
(22) Date of filing: 23.04.2003
(51) Int. Cl.: A61Q 5/00, A61Q 5/02, A61Q 5/12, A61K 8/90, A61K 8/89, A61K 8/898, A61K 8/892, A61K 8/894

(54) **CONDITIONING SHAMPOO COMPOSITION**
HAARPFLEGESHAMPOO
COMPOSITION POUR SHAMPOOING CONDITIONNEUR

(30) Priority: 10.05.2002 GB 0210791; 11.12.2002 GB 0228880
(43) Date of publication of application: 16.02.2005
(73) Proprietor: UNILEVER PLC, London EC4P 4BQ (GB); UNILEVER N.V., 3013 AL Rotterdam (NL)
(72) Inventor: DERICI, Leo, Unilever R & D Port Sunlight, Wirral, Merseyside CH63 3JW (GB); JENKINS, Paul David,2Unilever R & D Port Sunlight, Wirral, Merseyside CH63 3JW (GB); SHAW, Neil Scott, Unilever R & D Port Sunlight, Wirral, Merseyside CH63 3JW (GB); TAN-WALKER,Ruby L.B., Unilever R & D Port Sunlight, Wirral, Merseyside CH63 3JW (GB)
(74) Representative: Whaley, Christopher
(86) International application number: PCT/EP2003/004239
(87) International publication number: WO 2003/094874

(56) References cited:
- EP-A- 0 715 842
- WO-A-97/14405
- WO-A-98/50005
- US-A- 5 837 661
- US-A- 5 985 295
- US-A- 6 056 946

## Description

### Technical Field

The invention is concerned with rinse-off hair-conditioning compositions which are applied to the hair and then substantially rinsed away. It is particularly concerned with hair shampoo compositions, which both clean the hair and provide conditioning benefit to the hair. More specifically, it is concerned with improving the deposition of silicone conditioning oil onto the tip region of hair relative to the root region, from shampoo compositions which contain dispersed hydrophobic conditioning oil droplets and which are substantially free of cationic deposition polymers.

### Background and Prior Art

Compositions which provide a combination of cleansing and conditioning to the hair are well known in the art. Such shampoo or shower-gel compositions typically comprise one or more surfactants for shampooing or cleansing purposes and one or more conditioning agents. The purpose of the conditioning agent is to make the hair easier to comb when wet and more manageable when dry, e.g. less static and fly-away. Typically, these conditioning agents are water-insoluble oily materials, cationic polymers or cationic surfactants.

Amongst the most popular conditioning agents used in shampoo products are oily materials such as mineral oils, naturally occurring oils such as triglycerides and silicone polymers. These are generally present in the shampoo as dispersed hydrophobic emulsion droplets. Conditioning is achieved by the oily material being deposited onto the hair resulting in the formation of a film.

Conditioning compositions which provide conditioning only, without cleansing surfactants, are also well known in the art. Such compositions are generally applied to the hair after the cleansing composition has been rinsed away.

One method to improve deposition of conditioning oils onto hair is to use large droplets of oil. This method relies on physical contact between the hair and the droplets followed by the oil droplet wetting the hair surface and spreading.

Natural oils secreted by the sebaceous gland at the base of the hair lead to hair being more hydrophobic near the root rather than near the tip. This means that droplets deposited onto hair by the use of large droplets of oil are more likely to spread and form films on the hair at the base of the hair rather than near the tip of the hair, and this is found in practice.

Another method to enhance deposition of the conditioning oil droplets onto the hair is to employ a cationic deposition polymer in the composition. The use of such polymers is well known in the prior art.

The use of cationic polymers means that the oil droplets are flocculated with the cationic polymer on dilution of the shampoo when the hair is rinsed. This leads to indiscriminate deposition of the cationic polymer, conditioning oil and any other insoluble materials onto the hair. The presence of extraneous materials in addition to the conditioning oil can lead to dulling of the appearance of the hair (loss of shine) and also to a heavy feel to the hair (because of the presence of the cationic polymer).

Certain consumers find the effects arising from the two deposition methods described above to be undesirable in that they lead to the hair feeling greasy at the roots or heavy and dull.

US 3753916 discloses the use of cationic polymers as deposition aids for conditioning oils. Poloxamers are mentioned under the trade name Pluronic as optional ingredients.

WO 92/14440 discloses Pluronics (Poloxamers) in anti-dandruff shampoos as one of a class of 'synergisers' that unexpectedly enhance anti-dandruff efficacy.

WO 96/17590 discloses personal washing compositions containing lipids as the conditioning phase. The lipids are emulsified with a nonionic emulsifier. Poloxamers are listed as one of the possible polyalkoxy nonionic emulsifiers with HLB (hydrophile lipophile balance) in the range 1 to 15.

US 5100657 lists Pluronics (Poloxamers) as optional ingredients in hair conditioner compositions.

In attempts to overcome the problems in the prior art, it has been considered desirable to target the deposition of the conditioning oil droplets onto the tip regions of the hair in preference to the root regions, and much research has been carried out in this field of work. Although it would be desirable to make the surface of the oil droplets more hydrophilic, it had always been considered that the high levels of surfactant in shampoo compositions would dominate the surface chemistry and hydrophilicity of the oil droplets. Thus the conventional view is that irrespective of any additives added to the conditioning oil droplets, the shampoo surfactant would control the droplet hydrophilicity and deposition.

It has now surprisingly been found, that by blending certain types of surface active block copolymers of a high molecular weight with silicone conditioning oil emulsion droplets, enhanced deposition of the droplets onto the tip regions of hairs can be achieved. Although not wishing to be bound by the scientific reasoning underlying this phenomenon, it seems that surface active polymer remains at the droplet surface, even in the presence of other surfactant molecules from the shampoo, making the droplets more hydrophilic than the droplets in conventional oil droplet-containing shampoos. This leads to improved deposition of the droplets towards the more hydrophilic tip region of the hair.

### Summary of the invention

In a first aspect the invention provides a rinse-off an aqueous hair conditioning composition comprising :
a) from 1% to 50% by weight of a cleansing surfactant,
b) discrete, dispersed droplets comprising a water-insoluble silicone conditioning oil characterised in that the mean diameter of the droplets (D_{3,2}) is from 2 to 100 micrometres and
c) a surface active block copolymer with a mean molecular weight of 7000 unified atomic mass units or more comprising polyethyleneoxide blocks and polypropyleneoxide blocks, wherein each block consists of 2 or more ethylene oxide or propylene oxide monomer units and wherein the mean number propylene oxide monomer units in the block copolymer is 25 or more.

One preferred form of the surface active block copolymer is according to formula I: 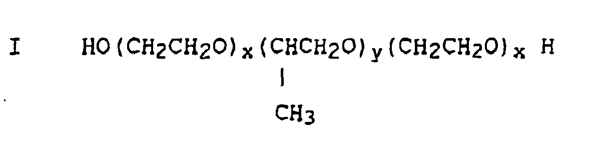 wherein the mean value of x is 4 or more and the mean value of y is 25 or more.

Another preferred form of the surface active block copolymer is according to formula II: 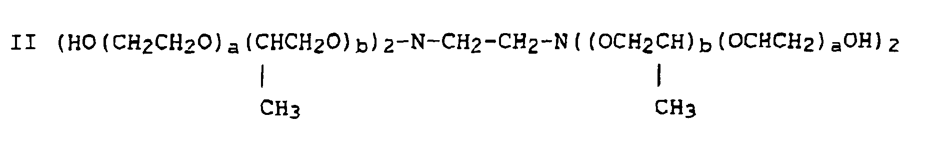 wherein the mean value of a is 2 or more and the mean value of b is 6 or more.

### Detailed Description

Compositions in accordance with the invention are formulated as compositions for the cleansing of hair and subsequent rinsing such as shampoos, cleansing mousses or shower gels. It is highly preferred that cleansing compositions according to the invention should contain less than 0.01% by weight of any cationic deposition polymer.

### Surface Active Polymer

An essential component of compositions according to the invention is a surface active block copolymer. This is a block copolymer based on polyethyleneoxide (EO) and polypropyleneoxide (PO) blocks. The molecular weight of the block copolymer is 7000 unified atomic mass units or more, more preferably 10000 or more, most preferably 12000 or more.

The mean molecular weight is suitably measured by determining the hydroxyl number for the polymer then transforming this into molecular weight. This corresponds to a number based mean molecular weight.

Each block of EO or PO polymer in the surface active block copolymer molecule suitably consists of 2 or more of the respective monomer units, preferably of 4 or more, more preferably of 8 or more, even more preferably of 25 of more and most preferably of 40 or more monomer units.

The surface active block copolymer molecule suitable comprises a mean number of 25 or more propylene oxide monomer units, preferably 35 or more, more preferably 45 or more and most preferably 60 or more.

Suitable EO/PO block copolymers according to formula I have the CTFA designation Poloxamer. These are commercially available under the trade name "Pluronic" from BASF.

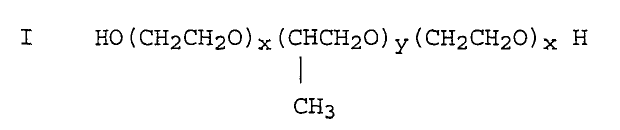

Suitably, the mean value of x in formula I is 4 or more, preferably 8 or more, more preferably 25 or more, yet more preferably 50 or more and most preferably 80 or more. Suitably, the mean value of y is 25 or more, preferably 35 or more, more preferably 45 or more and most preferably 60 or more.

Suitable EO/PO block copolymers according to formula II have the CFTA designation Poloxamine and are commercially available under the trade name "Tetronic" from BASF.

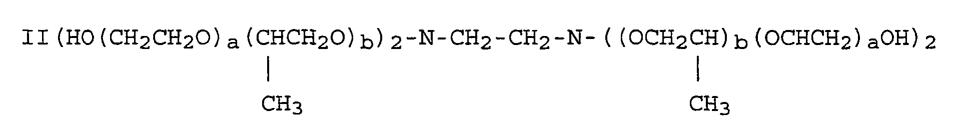

Suitably, the mean value of a is 2 or more, preferably 4 or more, more preferably 8 or more, even more preferably 25 or more and most preferably 40 or more. The mean value of b is suitably 6 or more, preferably 9 or more, more preferably 11 or more and most preferably 15 or more.

In formula I, the degree of polymerisation, x, is indicated as the same for each polyethyleneoxide block. This is also the case in formula II for a and b for the EO and PO blocks respectively. For the sake of clarity, it should be explained that these degrees of polymerisation are mean values and are approximately the same rather than identical for any particular formula. This is a result of the polymerisation methods used for production of the compounds and known to those skilled in the art of polymer synthesis.

Suitably, the level of surface active block copolymer is from 0.01% to 0.4% by weight of the composition, preferably from 0.02% to 0.3%, more preferably from 0.04% to 0.2%, even more preferably from 0.05% to 0.15%.

### Silicone Conditioning Oil

An essential component of compositions according to the invention is hydrophobic silicone conditioning oil. In order for such an oil to exist in discrete droplets in the compositions according to the invention, it must be water-insoluble. By water-insoluble is meant that the solubility in water at 25°C is 0.01% by weight or less.

It is essential that the D_{3,2} average particle diameter of the hydrophobic conditioning oil droplets in the composition is 2 micrometres or more, preferably 5 micrometres or more, and more preferably 8 micrometres or more. The average particle size of the oil droplets in the composition is 100 micrometres or less to prevent problems in stabilising the composition from separation of components.

Silicone D_{3,2} mean droplet diameter may be measured by means of a laser light scattering technique, for example using a 2600D Particle Sizer from Malvern Instruments.

The total amount of silicone conditioning oil present in the composition is preferably from 0.01% to 10 % by weight of the total composition more preferably from 0.3% to 5%, most preferably 0.5% to 3 %.

Suitable silicones for use as conditioning oils include polydiorganosiloxanes, in particular polydimethylsiloxanes which have the CTFA designation dimethicone. Also suitable for use compositions of the invention are polydimethyl siloxanes having hydroxyl end groups, which have the CTFA designation dimethiconol.

It is preferred if the silicone oil also comprises a functionalised silicone. Suitable functionalised silicones include, for example, amino-, carboxy-, betaine-, quaternary ammonium-, carbohydrate-, hydroxy- and alkoxy-substituted silicones. Preferably, the functionalised silicone contains multiple substitutions.

For the avoidance of doubt, as regards hydroxyl-substituted silicones, a polydimethylsiloxane merely having hydroxyl end groups (which have the CTFA designation dimethiconol) is not considered a functionalised silicone within the present invention. However, a polydimethylsiloxane having hydroxyl substitutions along the polymer chain is considered a functionalised silicone.

Preferred functionalised silicones are amino-functionalised silicones. Suitable amino functionalised silicones are described in EP 455,185 (Helene Curtis) and include trimethylsilylamodimethicone as depicted below, and are sufficiently water insoluble so as to be useful in compositions of the invention:

Si(CH₃)₃-O-[Si(CH₃)₂-O-]ₓ-[Si(CH₃)(R-NH-CH₂CH₂ NH₂)-O-]_{y}-Si(CH₃)₃

wherein x + y is a number from about 50 to about 500, and the weight percent amine functionality is from about 0.03% to about 8%, and wherein R is an alkylene group having from 2 to 5 carbon atoms. Preferably, the number x + y is in the from 100 to 300, and the weight percent amine functionality is from about 0.03% to 8%.

As expressed here, the weight percent amine functionality is measured by titrating a sample of the amino-functionalised silicone against alcoholic hydrochloric acid to the bromocresol green end point. The weight percent amine is calculated using a molecular weight of 45 (corresponding to CH₃- CH₂-NH₂).

Suitably, the weight percent amine functionality measured and calculated in this way is from 0.03% to 8%, preferably from 0.5% to 4%.

An example of a commercially available amino-functionalised silicone useful in the silicone component of the composition of the invention is DC-8566 available from Dow Corning (INCI name: dimethyl,methyl (aminoethylaminoisobutyl) siloxane). This has a weight percent amine functionality of about 1.4%.

By "amino functional silicone" is meant a silicone containing at least one primary, secondary or tertiary amine group, or a quaternary ammonium group. Examples of suitable amino functional silicones include: polysiloxanes having the CTFA designation "amodimethicone". Specific examples of amino functional silicones suitable for use in the invention are the aminosilicone oils DC-8220, DC-8166, DC-8466, and DC-8950-114 (all ex Dow Corning), and GE 1149-75, (ex General Electric Silicones). Suitable quaternary silicone polymers are described in EP-A-0 530 974. A preferred quaternary silicone polymer is K3474, ex Goldschmidt.

Another preferred functional silicone for use as a component in the hydrophobic conditioning oil is an alkoxy-substituted silicone. Such molecules are known as silicone copolyols and have one or more polyethyleneoxide or polypropyleneoxide groups bonded to the silicone polymer backbone, optionally through an alkyl linking group.

A non-limiting example of a type of silicone copolyol useful in compositions of the invention has a molecular structure according to the formula depicted below:

Si(CH₃)₃[O-Si(CH₃)(A)]ₚ-[O-Si(CH₃)(B)]_{q}-O-Si(CH₃)₃

In this formula, A is an alkylene chain with from 1 to 22 carbon atoms, preferably 4 to 18, more preferably 10 to 16. B is a group with the structure: -(R)-(EO)ᵣ(PO)ₛ-OH wherein R is a linking group, preferably an alkylene group with 1 to 3 carbon atoms. Preferably R is -(CH₂)₂-. The mean values of r and s are 5 or more, preferably 10 or more, more preferably 15 or more. It is preferred if the mean values of r and s are 100 or less. In the formula, the value of p is suitably 10 or more, preferably 20 or more, more preferably 50 or more and most preferably 100 or more. The value of q is suitably from 1 to 20 wherein the ratio p/q is preferably 10 or more, more preferably 20 or more. The value of p + q is a number from 11 to 500, preferably from 50 to 300.

Suitable silicone copolyols have an HLB of 10 or less, preferably 7 or less, more preferably 4 or less. A suitable silicone copolyol material is DC5200, known as Lauryl PEG/PPG - 18/18 methicone (INCI name), available from Dow Corning.

Hydrophile/Lipophile balance or HLB is a well known parameter used by those skilled in the art to characterise surface active molecules and emulsifiers.

Suitable methods for the experimental determination of HLB are in Griffin W.C, Journal of the Society of Cosmetic Chemists, volume 1 page 311 (1949). The commercially available silicone copolyols are supplied along with a value of their HLB by Dow Corning.

It is preferred to use a combination of functional and non-functional silicones as the conditioning oil. Preferably these are blended into common droplets prior to incorporation into compositions according to the invention.

The viscosity of the silicone oil blend measured in isolation from the rest of the composition (i.e. not the viscosity of any pre-formed emulsion, but of the silicone blend forming the hydrophobic conditioning oil) is suitably from 1,000 to 2,000,000 mm²sec⁻¹ at 25 °C, preferably from 5,000 to 1,000,000, more preferably from 10,000 to 500,000, most preferably from 50,000 to 300,000. Suitable methods for measuring the kinematic viscosity of silicone oils are known to those skilled in the art, e.g. capillary viscometers.

For high viscosity silicones, a constant stress rheometer can be used to measure viscosity.

### Cationic Deposition Polymer

A cationic deposition polymer is often used in hair treatment compositions, for enhancing conditioning performance. However this leads to problems arising from the cationic polymer also remaining adhered to the hair, which can, in some cases, lead to a dirty, sticky feel to the hair some hours after use of the cleansing and conditioning composition for some consumers.

Such cationic polymers may be a homopolymer or be formed from two or more types of monomers. The molecular weight of the polymer will generally be between 5 000 and 10 000 000 unified atomic mass units, typically at least 10 000 and preferably from 100 000 to 2 000 000. The polymers will have cationic nitrogen containing groups such as quaternary ammonium or protonated amino groups, or a mixture thereof.

It is highly preferred that compositions according to the invention should contain less than 0.04% by weight of such a cationic deposition polymer, more preferably less than 0.02%, even more preferably less than 0.01%. It is most preferred that such polymers are absent from the composition.

### Preparation of Compositions

One method for preparing compositions according to the invention is to add a silicone conditioning oil, along with the other components comprising the hair treatment composition, followed by suitable mixing of the composition in order to ensure that the blend is dispersed as droplets of a suitable size.

However it is preferred if the hydrophobic silicone conditioning oil is first formed into an aqueous emulsion prior to incorporation into the hair treatment composition. Thus another aspect of the invention is a method for preparing an aqueous hair conditioning composition comprising the steps of :
i) preparing a solution comprising water and the surface active block copolymer,
ii) adding a silicone conditioning oil to the solution,
iii) forming the solution and the silicone conditioning oil into an oil-in-water emulsion by high-shear mixing,
iv) dispersing the oil-in-water emulsion comprising the block copolymer into a hair conditioning composition.

Another preferred method for preparing a hair conditioning composition according to the invention comprises the steps of:
i) preparing an oil-in-water emulsion of a silicone conditioning oil,
ii) dispersing the surface active block copolymer into the emulsion,
iii) dispersing the oil-in-water emulsion comprising the block copolymer into a hair conditioning composition.

Suitable emulsifiers for use in the preparation of the aqueous emulsion are well known in the art and include anionic, cationic, zwitterionic, amphoteric and nonionic surfactants, and mixtures thereof. Examples of anionic surfactants used as emulsifiers for the silicone particles are alkylarylsulphonates, e.g., sodium dodecylbenzene sulphonate, alkyl sulphates e.g., sodium lauryl sulphate, alkyl ether sulphates, e.g., sodium lauryl ether sulphate nEO, where n is from 1 to 20, alkylphenol ether sulphates, e.g., octylphenol ether sulphate nEO where n is from 1 to 20, and sulphosuccinates, e.g., sodium dioctylsulphosuccinate.

Examples of nonionic surfactants suitable for use as emulsifiers for the silicone droplets are alkylphenol ethoxylates, e.g., nonylphenol ethoxylate nEO, where n is from 1 to 50 and alcohol ethoxylates, e.g., lauryl alcohol nEO, where n is from 1 to 50, ester ethoxylates, e.g., polyoxyethylene monostearate where the number of oxyethylene units is from 1 to 30.

A preferred process for preparing oil-in-water emulsions of the mixed silicone droplets which can then be incorporated in the hair treatment compositions involves use of a mixer.

Depending upon the viscosities of components of the silicone mixture a suitable mixer should be chosen so as to provide sufficient shear to give the required final particle size of the emulsion. Examples of suitable benchtop mixers spanning the range of necessary shear are Heidolph RZR2100, Silverson L4R and Ystral X10/20-750. Other mixers of similar specification are well known to those skilled in the art and can also be used in this application. Equally it is possible to manufacture oil-in-water emulsions of this description on larger scale mixers which offer similar shear regimes to those described above.

Preferably, the mixer is also capable of having the temperature of mixing controlled, e.g. it comprises a jacket through which a heat transfer fluid can be circulated.

It is preferred if the aqueous phase of the emulsion contains a polymeric thickening agent to prevent phase separation of the emulsion after preparation. Preferred thickening agents are cross-linked polyacrylates, cellulosic polymers or derivatives of cellulosic polymers.

### Cleansing Surfactant

Compositions according to the invention will comprise one or more cleansing surfactants which are cosmetically acceptable and suitable for topical application to the hair. Further surfactants may be present as an additional ingredient if sufficient for cleansing purposes is not provided by the emulsifier for the water-insoluble oily component. It is preferred that shampoo compositions of the invention comprise at least one further surfactant (in addition to any used as emulsifying agent for the silicone component) to provide a cleansing benefit.

Suitable cleansing surfactants, which may be used singularly or in combination, are selected from anionic, amphoteric and zwitterionic surfactants, and mixtures thereof. The cleansing surfactant may be the same surfactant as the emulsifier, or may be different.

The total amount of surfactant (including any co-surfactant, and/or any emulsifier) in compositions of the invention is generally from 1 to 50, preferably from 2 to 40, more preferably from 10 to 25 percent by weight of the composition.

### Anionic Cleansing Surfactant

Shampoo compositions according to the invention will typically comprise one or more anionic cleansing surfactants which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates, and alpha-olefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Typical anionic cleansing surfactants for use in shampoo compositions of the invention include sodium oleyl succinate, ammonium lauryl sulphosuccinate, ammonium lauryl sulphate, sodium dodecylbenzene sulphonate, triethanolamine dodecylbenzene sulphonate, sodium cocoyl isethionate, sodium lauryl isethionate and sodium N-lauryl sarcosinate. The most preferred anionic surfactants are sodium lauryl sulphate, sodium lauryl ether sulphate(n)EO, (where n is from 1 to 3), ammonium lauryl sulphate and ammonium lauryl ether sulphate(n)EO, (where n is from 1 to 3).

Mixtures of any of the foregoing anionic cleansing surfactants may also be suitable.

The total amount of anionic cleansing surfactant in shampoo compositions of the invention is generally from generally from 0.5 to 45, preferably from 1.5 to 35, more preferably from 5 to 20 percent by weight of the composition.

For the sake of clarity, the surface active block copolymer is not considered here to be a cleansing surfactant.

### Co-surfactant

The composition can include co-surfactants, to help impart aesthetic, physical or cleansing properties to the composition.

A preferred example is an amphoteric or zwitterionic surfactant, which can be included in an amount ranging from 0 to about 8, preferably from 1 to 4 percent by weight.

Examples of amphoteric and zwitterionic surfactants include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine and preferably lauryl betaine, cocamidopropyl betaine and sodium cocamphopropionate.

Another preferred example is a nonionic surfactant, which can be included in an amount ranging from 0 to 8, preferably from 2 to 5 percent by weight of the composition.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or diethanolamide and coco mono-isopropanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)ₙ

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n is from about 1.1 to about 2.

Most preferably the value of n is from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the C₁₀-C₁₈ N-alkyl (C₁-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

The composition according to the invention can also optionally include one or more cationic co-surfactants included in an amount ranging from 0.01 to 10, more preferably from 0.05 to 5, most preferably from 0.05 to 2 percent by weight of the composition.

A preferred blend of surfactants comprises a mixture of ammonium lauryl ether sulfate, ammonium lauryl sulfates, PEG 5 cocamide and cocomide MEA (CTFA designations).

### Suspending Agents

Preferably, the compositions according to the invention further comprise from 0.1 to 10 percent by weight, preferably from 0.6% to 6%, of a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives. Polyacrylic acid is available commercially as Carbopol 420, Carbopol 488 or Carbopol 493. Polymers of acrylic acid cross-linked with a polyfunctional agent, known as cross-linked polyacrylates, may also be used, they are available commercially as Carbopol 910, Carbopol 934, Carbopol 940, Carbopol 941 and Carbopol 980. An example of a suitable copolymer of a carboxylic acid containing a monomer and acrylic acid esters is Carbopol 1342. All Carbopol (trade mark) materials are available from Goodrich. The CTFA designation for cross-linked polyacrylates is Carbomer.

Suitable cross-linked polymers of acrylic acid and acrylate esters are Pemulen TR1 or Pemulen TR2. A suitable heteropolysaccharide gum is xanthan gum, for example that available as Kelzan mu.

### Adjuvants

The compositions of the present invention may also contain adjuvants suitable for hair care. Generally such ingredients are included individually at a level of up to 2%, preferably up to 1 % by weight of the total composition.

Among suitable hair care adjuvants, are natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts. A particularly preferred combination of natural hair root nutrients for inclusion in compositions of the invention is isoleucine and glucose. A particularly preferred amino acid nutrient is arginine. Another suitable adjuvant is glycolic acid.

### Optional Ingredients

Compositions of this invention may contain any other ingredient normally used in hair treatment formulations. These other ingredients may include viscosity modifiers, preservatives, colouring agents, polyols such as glycerine and polypropylene glycol, chelating agents such as EDTA, antioxidants, fragrances, antimicrobials and sunscreens. Each of these ingredients will be present in an amount effective to accomplish its purpose. Generally these optional ingredients are included individually at a level of up to 5% by weight of the total composition.

### Mousses

Hair treatment cleansing and conditioning compositions in accordance with the invention may also take the form of aerosol foams (mousses) in which case a propellant is included in the composition. This agent is responsible for expelling the other materials from the container and forming the hair mousse character.

The propellant gas can be any liquefiable gas conventionally used for aerosol containers. Examples of suitable propellants include dimethyl ether, propane, n-butane and isobutane, used singly or in admixture.

The amount of the propellant gases is governed by normal factors well known in the aerosol art. For hair mousses, the level of propellant is generally from 3 to 30, preferably from 5 to 15 % by weight of the total composition.

### Mode of Use

The compositions of the invention are primarily intended for topical application to the hair and/or scalp and or skin of a human subject as rinse-off treatments to clean the hair or body while improving hair fibre surface properties such as smoothness, softness, manageability, cuticle integrity, and shine. Typically such compositions are known in the art as shampoos, cleansing mousses or shower gels.

In particular, compositions according to the invention are used for improving the deposition of silicone conditioning oil onto the tip region of hair relative to the root region of hair.

Thus one aspect of the invention comprises a method of cleaning and conditioning hair by applying compositions according to the invention followed by rinsing.

The invention is further demonstrated with reference to the following, non-limiting examples:

### Examples

Examples 1 to 9 are examples according to the invention. Examples A to C are comparative examples.

**Table 1**

| **Ingredient** | **Trade Name** | **Supplier** | **Example A weight %** | **Examples B,C and 1-6 weight %** |
|---|---|---|---|---|
| Sodium laureth (2 EO) sulphate | Empicol ESB70 | Albright & Wilson | 16 | 16 |
| Coco amidopropyl betaine | Tegobetaine CK | Goldschmidt | 2 | 2 |
| PDMS fluid (760,000 mm² sec⁻¹) | DC200 | Dow Corning | 1 | 1 |
| block copolymer | Various (see table 2) | | - | 0.05 |
| Sodium Chloride | Salt | BDH | 1 | 1 |
| Water | - | - | To 100 | To 100 |

Compositions in table 1 were prepared as described above by forming an aqueous emulsion of the DC200 using a suitable nonionic emulsifier and the block copolymer (when present as indicated in the table). The subsequent emulsion was then blended with the other ingredients except salt. The salt was finally added to thicken the composition.

### Test Method

0.25g/5 cm switches of tip hair which had been cleaned with a solution of 14% SLES 2EO and 2% cocoamidopropyl betaine in water followed by extensive rinsing, were used for this experiment. The test shampoo was diluted to 1 in 10 by weight with distilled water and stirred throughout with a magnetic stirrer. 5 switches were placed in one half of a petri dish. 1.5 mls of diluted shampoo was placed along the length of the switches which were then agitated in the dish for 30 seconds, followed by a rinse for 30 seconds under tap water (12° French hard) at 40°C, with a flow rate set at 3-4 litres per minute. The washing process using the test shampoo solution was repeated followed again by rinsing. The switches were then allowed to dry naturally at 25° C and a relative humidity of 45 to 60%.

The same sequence of experimentation was carried out for 0.25g/5 cm samples of root hair.

The amount of silicone deposited on the hair samples was measured using X-ray fluorescence spectrometry (measured in parts per million (ppm) of silicon).

### Analysis results

The absolute selectivity expressed as a percentage is the ratio of silicon ppm on the tip samples to silicon ppm on the root samples multiplied by 100.

The absolute selectivity calculated for Example A (without block copolymer) was then subtracted from the absolute selectivity calculated for each of the other Example formulations to derive a targeting score. The results are shown below in Table 2. A targeting score of 5% or less is considered to be of no significant value.

**Table 2**

| **Example** | **Polymeric emulsifier** | **x or a** | **y or b** | **Molecular Weight** | **Targeting score(%)** |
|---|---|---|---|---|---|
| A | None (DC200 benchmark) | | | - | 0 |
| B | Pluronic F38 | 42 | 16 | 4700 | 0 |
| C | Pluronic P84 | 19 | 39 | 4200 | 5 |
| 1 | Pluronic F68 | 76 | 30 | 8400 | 12 |
| 2 | Pluronic F88 | 104 | 39 | 11400 | 10 |
| 3 | Pluronic F77 | 52 | 35 | 6600 | 9 |
| 4 | Pluronic F98 | 123 | 47 | 13000 | 9 |
| 5 | Pluronic F127 | 99 | 69 | 12600 | 24 |
| 6 | Tetronic T1307 | 72 | 23 | 18000 | 38 |

The Pluronic and Tetronic samples are commercially available from BASF under the trade names as listed in the table.

For the Pluronic and Tetronic block copolymers in the examples, the last digit in the sample code in table 2 reveals the weight percent polyethyleneoxide in the block copolymer (i.e. P84 comprises 40% of polyethyleneoxide and T1307 comprises 70%).

**Table 3**

| **Ingredient** | **Trade Name** | **Supplier** | **Examples** | | | |
|---|---|---|---|---|---|---|
| | | | **A** | **7** | **8** | **9** |
| Sodium laureth (2 EO) sulphate | Empicol ESB70 | Albright & Wilson | 16 | 16 | 16 | 16 |
| Coco amidopropyl betaine | Tegobetaine CK | Goldschmidt | 2 | 2 | 2 | 2 |
| PDMS fluid (60,000 mm²sec⁻¹) | DC200 | Dow Corning | 1 | 1 | 0.5 | - |
| PDMS fluid 300,000 mm² sec⁻¹ | DC200 | Dow Corning | - | - | - | .67 |
| Lauryl PEG/PPG - 18/18 methicone | DC5200 | Dow Corning | - | - | 0.5 | - |
| Dimethyl,methyl (aminoethylaminoi sobutyl) siloxane | DC8566 | Dow Corning | - | - | - | 0.33 |
| block copolymer | Pluronic F127 | BASF | - | 0.05 | 0.05 | 0.05 |
| Sodium Chloride | Salt | BDH1 | 1 | 1 | 1 | 1 |
| Water | - | - | to 100 | to 100 | to 100 | to 100 |

DC5200 is a silicone copolyol which is commercially available from Dow Corning which has an HLB of considerably less than 10.

DC8566 is an amodimethicone (amino-functionalised silicone) which is commercially available from Dow Corning.

The examples in table 3 were prepared as for the examples in table 1, but for examples 8 and 9, the functionalised silicones were blended into the PDMS fluid prior to emulsification to ensure that the two silicones were present in common droplets.

**Table 4**

| **Example** | **Targeting score (%)** |
|---|---|
| A | 0 |
| 7 | 24 |
| 8 | 166 |
| 9 | 78 |

The results in table 4 show highly significant hair tip targeting for examples 7 to 9, which are according to the invention, compared to example A which is not according to the invention.

## Claims

1. A rinse-off aqueous hair conditioning composition comprising;
a) from 1% to 50% by weight of a cleansing surfactant,
b) discrete, dispersed droplets comprising a water-insoluble silicone conditioning oil **characterised in that** the mean diameter of the droplets (D_{3,2}) is from 2 to 100 micrometres and
c) a surface active black copolymer with a mean molecular weight of 7000 unified atomic mass units or more comprising polyethyleneoxide blocks and polypropyleneoxide blocks, wherein each block consists of 2 or more ethylene oxide or propylene oxide monomer units and wherein the mean number propylene oxide monomer unite in the block copolymer is 25 or more.

2. A composition according to claim 1 wherein the surface active block polymer is according to formula I: 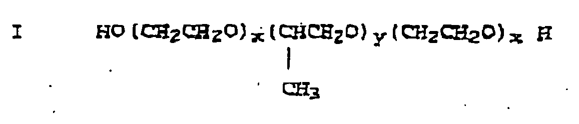 wherein the mean value of x is 4 or more and the mean value of y is 25 or more.

3. A composition according to claim 1 wherein the surface active block copolymer is according to formula II: 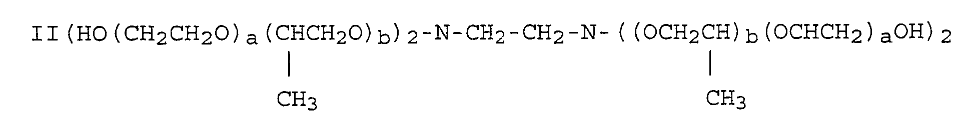 wherein the mean value of a is 2 or more and the mean value of b is 6 or more.

4. A composition according to any preceding claim comprising from 0.01% to 0.4% by weight of surface active block copolymer.

5. A composition according to any preceding claim wherein the silicone conditioning oil has a viscosity from 5000 to 1000000 mm²sec⁻¹ at 25° C.

6. A composition according to any preceding claim wherein the silicone oil comprises a functionalised silicone.

7. A composition according to claim 6 wherein the functionalised silicone is a silicone copolyol with an HLB of 10 or less.

8. A composition according to claim 6, wherein the functionalised silicone is an amino-functionalised silicone.

9. A composition according to claim 8 wherein the amino-functionalised silicone has a weight percent amino functionality from 0.03 to 8, preferably from 0.5 to 4 percent.

10. A composition according to any preceding claim wherein the composition comprises less than 0.01% by weight of a cationic deposition polymer.

11. A composition according to any preceding claim wherein the cleansing surfactant is selected from the group consisting of anionic, amphoteric, zwitterionic and nonionic surfactants and mixtures thereof.

12. A method for preparing an aqueous hair conditioning composition according to any preceding claim comprising the steps of :
i) preparing a solution comprising water and the surface active block copolymer,
ii) adding a silicone conditioning oil to the solution,
iii) forming the solution and the silicone conditioning oil into an oil-in-water emulsion by high-shear mixing,
iv) dispersing the oil-in-water emulsion comprising the block copolymer into a hair conditioning composition.

13. A method for preparing a hair conditioning composition according to claim 1 comprising the steps of:
i) preparing an oil-in-water emulsion of a silicone conditioning oil,
ii) dispersing the surface active block copolymer into the emulsion,
iii) dispersing the oil-in-water emulsion comprising the block copolymer into a hair conditioning composition.

14. The use of a composition according to any one of claims 1 to 11 for cleaning and conditioning hair.

15. A method of cleaning and conditioning hair by applying a composition according to any one of claims 1 to 11 followed by rinsing.

16. The use of a composition according to any of claims 1 to 11 for improving the deposition of silicone conditioning oil onto the tip region of hair relative to the root region of hair.

## Patentansprüche

1. Ausspülbare wässerige Haarpflegezusammensetzung, umfassend
a) 1 bis 50 Gew.-% eines reinigenden Tensids,
b) voneinander getrennte, dispergierte Tröpfchen, die ein wasserunlösliches Silikonpflegeöl umfassen, **dadurch gekennzeichnet, dass** der mittlere Durchmesser der Tröpfchen (D_{3,2}) 2 bis 100 µm beträgt, und
c) ein oberflächenaktives Blockcopolymer mit einem mittleren Molekulargewicht von 7000 atomaren Masseneinheiten oder mehr, umfassend Polyethylenoxidblöcke und Polypropylenoxidblöcke, wobei jeder Block aus 2 oder mehr Ethylenoxid- oder Propylenoxidmonomereinheiten besteht und wobei die mittlere Anzahl an Propylenoxidmonomereinheiten im Blockcopolymer 25 oder mehr beträgt.

2. Zusammensetzung nach Anspruch 1, in der das oberflächenaktive Blockcopolymer der Formel I entspricht 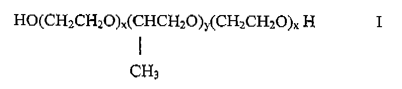 wobei der mittlere Wert von x 4 oder mehr ist und der mittlere Wert von y 25 oder mehr ist.

3. Zusammensetzung nach Anspruch 1, in der das oberflächenaktive Blockcopolymer der Formel II entspricht 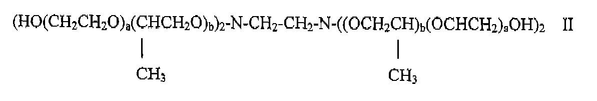 wobei der mittlere Wert von a 2 oder mehr ist und der mittlere Wert von b 6 oder mehr ist.

4. Zusammensetzung nach einem der vorstehenden Ansprüche, umfassend 0,01 bis 0,4 Gew.-% eines oberflächenaktiven Blockcopolymers.

5. Zusammensetzung nach einem der vorstehenden Ansprüche, in der das Silikonpflegeöl eine Viskosität von 5.000 bis 1.000.000 mm²sec⁻¹ bei 25°C hat.

6. Zusammensetzung nach einem der vorstehenden Ansprüche, in der das Silikonöl ein funktionalisiertes Silikon umfasst.

7. Zusammensetzung nach Anspruch 6, in der das funktionalisierte Silikon ein Silikoncopolyol mit einem HLB-Wert von 10 oder weniger ist.

8. Zusammensetzung nach Anspruch 6, in der das funktionalisierte Silikon ein aminofunktionalisiertes Silikon ist.

9. Zusammensetzung nach Anspruch 8, in der das aminofunktionalisierte Silikon eine Aminofunktionalität in Gew.-% von 0,03 bis 8, vorzugsweise von 0,5 bis 4 % hat.

10. Zusammensetzung nach einem der vorstehenden Ansprüche, wobei die Zusammensetzung weniger als 0,01 Gew.-% eines kationischen Abscheidungspolytners umfasst.

11. Zusammensetzung nach einem der vorstehenden Ansprüche, wobei das reinigende Tensid aus der aus anionischen, amphoteren, zwitterionischen oder nichtionischen Tensiden und deren Gemischen bestehenden Gruppe ausgewählt wird.

12. Verfahren zur Herstellung einer wässerigen Haarpflegezusammensetzung nach einem der vorstehenden Ansprüche mit den Schritten:
i) Herstellen einer Lösung, die Wasser und das oberflächenaktive Blockcopolymer umfasst,
ii) Zugeben eines Silikonpflegeöls zu der Lösung,
iii) Verarbeiten der Lösung und des Silikonpflegeöls zu einer Öl-in-Wasser-Emulsion durch Mixen mit hoher Scherung,
iv) Dispergieren der das Blockcopolymer umfassenden Öl-in-Wasser-Emulsion zu einer Haarpflegezusammensetzung.

13. Verfahren zur Herstellung einer Haarpflegezusammensetzung nach Anspruch 1 mit den Schritten:
i) Herstellen einer Öl-in-Wasser-Emulsion eines Silikonpflegeöls,
ii) Dispergieren des oberflächenaktiven Blockcopolymers in die Emulsion,
iii) Dispergieren der das Blockcopolymer umfassenden Öl-in-Wasser-Emulsion in eine Haarpflegezusammensetzung.

14. Verwendung einer Zusammensetzung nach einem der Ansprüche 1 bis 11 zum Waschen und Pflegen von Haaren.

15. Verfahren zum Waschen und Pflegen von Haaren durch Aufbringen einer Zusammensetzung nach einem der Ansprüche 1 bis 11 und anschließendes Ausspülen.

16. Verwendung einer Zusammensetzung nach einem der Ansprüche 1 bis 11 zur Verbesserung der Abscheidung von Silikonpflegeöl auf den Spitzenbereich des Haares relativ zum Wurzelbereich des Haares.

## Revendications

1. Composition aqueuse pour shampooing conditionneur s'éliminant par rinçage, comprenant :
a) de 1 % à 50 % en poids d'un tensioactif de lavage,
b) des gouttelettes dispersées, discrètes, comprenant une huile de conditionneur à la silicone insoluble dans l'eau, **caractérisées en ce que** le diamètre moyen des gouttelettes (D₃,₂) est de 2 à 100 micromètres, et
c) un copolymère séquencé tensioactif ayant un poids moléculaire moyen de 7 000 unités de masse atomique unifiées ou plus comprenant des séquences d'oxyde de polyéthylène et des séquences d'oxyde de polypropylène, dans lequel chaque séquence se compose de 2 ou davantage d'unités monomères d'oxyde d'éthylène ou d'oxyde de propylène et dans lequel le nombre moyen d'unités monomères d'oxyde de propylène dans le copolymère séquencé est de 25 ou plus.

2. Composition selon la revendication 1, dans laquelle le polymère séquencé tensioactif est représenté par la formule I : 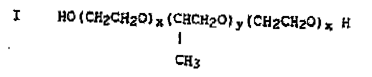 dans laquelle la valeur moyenne de x est de 4 ou plus et la valeur moyenne de y est de 25 ou plus.

3. Composition selon la revendication 1, dans laquelle le copolymère séquencé tensioactif est représenté par la formule II : 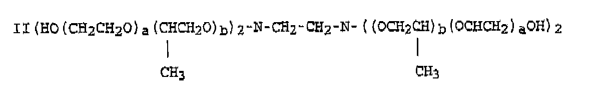 dans laquelle la valeur moyenne de a est de 2 ou plus et la valeur moyenne de b est de 6 ou plus.

4. Composition selon l'une quelconque des revendications précédentes, comprenant de 0,01 % à 0,4 % en poids de copolymère séquencé tensioactif.

5. Composition selon l'une quelconque des revendications précédentes, dans laquelle l'huile de conditionneur à la silicone a une viscosité de 5 000 à 1 000 000 mm²sec⁻¹ à 25° C.

6. Composition selon l'une quelconque des revendications précédentes, dans laquelle l'huile de silicone comprend une silicone fonctionnalisée.

7. Composition selon la revendication 6, dans laquelle la silicone fonctionnalisée est un copolyol de silicone avec un équilibre hydrophile - lipophile (HLB) de 10 ou moins.

8. Composition selon la revendication 6, dans laquelle la silicone fonctionnalisée est une silicone fonctionnalisée par un amino.

9. Composition selon la revendication 8, dans laquelle la silicone fonctionnalisée par un amino a un pourcentage en poids de la fonction amino de 0,03 à 8, de préférence de 0,5 à 4 pour cent.

10. Composition selon l'une quelconque des revendications précédentes, dans laquelle la composition comprend moins de 0,01 % en poids d'un polymère à dépôt cationique.

11. Composition selon l'une quelconque des revendications précédentes, dans laquelle le tensioactif de lavage est choisi dans le groupe consistant en des tensioactifs anioniques, amphotères, zwitterioniques et non ioniques et des mélanges de ces derniers.

12. Procédé pour préparer une composition aqueuse pour shampooing conditionneur selon l'une quelconque des revendications précédentes, comprenant les étapes consistant à :
i) préparer une solution comprenant de l'eau et le copolymère séquencé tensioactif,
ii) ajouter une huile de conditionneur à la silicone à la solution,
iii) former la solution et l'huile de conditionneur à la silicone en une émulsion d'huile dans l'eau par mélange à fort cisaillement,
iv) disperser l'émulsion d'huile dans l'eau comprenant le copolymère séquencé dans une composition pour shampooing conditionneur.

13. Procédé pour préparer une composition pour shampooing conditionneur selon la revendication 1, comprenant les étapes consistant à :
i) préparer une émulsion d'huile dans l'eau d'une huile de conditionneur à la silicone,
ii) disperser le copolymère séquencé tensioactif dans l'émulsion,
iii) disperser l'émulsion d'huile dans l'eau comprenant le copolymère séquencé dans une composition pour shampooing conditionneur.

14. Utilisation d'une composition selon l'une quelconque des revendications 1 à 11 pour laver et conditionner les cheveux.

15. Procédé pour laver et conditionner les cheveux par application d'une composition selon l'une quelconque des revendications 1 à 11, suivie du rinçage.

16. Utilisation d'une composition selon l'une quelconque des revendications 1 à 11 pour améliorer le dépôt d'huile de conditionneur à la silicone sur la région de la pointe du cheveu relativement à la région de la racine du cheveu.
